Europäisches Patentamt

European Patent Office

Office européen des brevets

(19)

(11) Veröffentlichungsnummer: **0 012 907**
**B1**

(12) # EUROPÄISCHE PATENTSCHRIFT

(45) Veröffentlichungstag der Patentschrift:
**09.12.81**

(51) Int. Cl.³: **C 07 C 29/88,** C 07 C 29/76,
C 07 C 31/18

(21) Anmeldenummer: **79105043.8**

(22) Anmeldetag: **10.12.79**

(54) **Verfahren zur Reinigung von niedermolekularen Polyhydroxylverbindungen.**

(30) Priorität: **20.12.78 DE 2855038**

(43) Veröffentlichungstag der Anmeldung:
**09.07.80 Patentblatt 80/14**

(45) Bekanntmachung des Hinweises auf die Patenterteilung:
**09.12.81 Patentblatt 81/49**

(84) Benannte Vertragsstaaten:
**BE CH DE FR GB IT**

(56) Entgegenhaltungen:
DE-A-2714084
DE-B-1065399
DE-C-967552
FR-A-930849
FR-A-2363537
FR-A-2390412
GB-A-686254
GB-A-877643
GB-A-1479880
US-A-3076854

(73) Patentinhaber: **BAYER AG, Zentralbereich Patente,
Marken und Lizenzen, D-5090 Leverkusen 1, Bayerwerk
(DE)**

(72) Erfinder: **Klinkmann, Kurt, Stephan-Lochner-Strasse 35,
D-5090 Leverkusen 17 (DE)**
Erfinder: **Wambach, Raimund, Dr.,
Berta-von-Suttner-Strasse 65, D-5090 Leverkusen 1 (DE)**

ACTORUM AG.

Verfahren zur Reinigung von niedermolekularen Polyhydroxylverbindungen

Die vorliegende Erfindung betrifft ein Verfahren zur Reinigung von niedermolekularen Polyhydroxylverbindungen.

Es ist bekannt, niedermolekulare Polyhydroxylverbindungen beispielsweise durch Kondensation von Formaldehyd in Gegenwart von Bleiverbindungen (DE-OS 27 14 084; DE-OS 27 21 186) bzw. von Calciumverbindungen (DE-OS 27 21 186) herzustellen.

Niedermolekulare Polyhydroxylverbindungen, die beispielsweise durch die genannten Verfahren hergestellt werden können und die gegebenenfalls zur Reduktion von Carbonylgruppen zu Hydroxylgruppen hydriert werden können, stellen wertvolle Kettenverlängerer und Vernetzer für Polyurethankunststoffe dar (DE-OS 27 14 084). Sie können ferner durch Veresterung mit langkettigen, aliphatischen Monocarbonsäuren oder durch Oxäthylierung zu nichtionogenen oberflächenaktiven Verbindungen umgesetzt werden, wie dies analog bei K. Lindner, «Tenside» Band III, Wissenschaftliche Verlagsgesellschaft Stuttgart 1964, Seite 2336 beschrieben ist.

Für die genannten Anwendungen werden gereinigte niedermolekulare Polyhydroxylverbindungen benötigt. Beispielsweise ist die Entfernung von Calcium- und/oder Bleiverbindungen, die aus dem Herstellungsverfahren in den Lösungen von niedermolekularen Polyhydroxylverbindungen enthalten sind, im Anschluss an die Kondensation von Formaldehyd bzw. vor oder nach einer gegebenenfalls durchgeführten Hydrierung erforderlich.

Zur Reinigung von niedermolekularen Polyhydroxylverbindungen von Bleiverbindungen ist deren Ausfällung mit Schwefelsäure als Sulfat beschrieben (DE-OS 27 14 084).

Weiterhin ist aus US 3 076 854 die Reinigung von Trimethylolpropan von unerwünschtem Metallformiat, beispielsweise Na-formiat, und von unerwünschten Polyhydroxylverbindungen bekannt. Die Polyhydroxylverbindungen werden hierbei durch Erhitzen in Gegenwart von Methanol und Mineralsäuren, wie Salzsäure, Schwefelsäure oder Phosphorsäure, hydrolysiert. Anschliessend werden die ionischen Bestandteile dieses Gemisches an Anion- und Kationenaustauschern entfernt. In diesem Verfahren werden demnach Methanol und Mineralsäure nicht zur Ausfällung von Stoffen aus dem zu reinigenden Gemisch, sondern nur zur Hydrolyse eingesetzt. Sämtliche Ionen werden nur durch Ionenaustausch entfernt, wozu unwirtschaftlich grosse Austauscherkapazitäten erforderlich sind. Eine Berücksichtigung von komplizierten Gemischen, die beispielsweise noch Hydroxyaldehyde und Hydroxyketone enthalten und dadurch die Löslichkeitsverhältnisse von auszufällenden Ionen, beispielsweise von komplexbildenden mehrwertigen Katalysatorionen, beeinflussen, ist in dieser US-PS nicht vorgesehen.

In GB 1 479 880 wird beschrieben, aus einer wässrigen Glycerinlösung, die jedoch keine Hydroxyaldehyde und keine Hydroxyketone enthält, den grössten Teil der Na-Ionen durch Zugabe von $H_2SO_4$ und Methanol abzuscheiden.

Weiter ist es aus GB 686 254 bekannt, aus einer Pentaerythritlösung Calciumionen entweder mit einem Ionenaustauscher oder mit überschüssiger $H_2SO_4$ zu entfernen, wobei im letzteren Fall die zur Fällung nicht benötigte $H_2SO_4$ dann mit Bariumcarbonat gefällt wird.

Es wurde nun ein Verfahren zur Reinigung von wässrigen Lösungen von niedermolekularen Polyhydroxylverbindungen, die Calcium- oder Bleiverbindungen oder ein Gemisch von Calcium- und Bleiverbindungen neben Ameisensäure enthalten, durch Entfernung von Calcium- und/oder Bleiverbindungen sowie der Ameisensäure durch Mitverwendung von Ionenaustauscher- und Fällungsmethoden gefunden, das dadurch gekennzeichnet ist, dass man diesen Lösungen Methanol zufügt, eine den zu entfernenden Ionen äquivalente Menge Fällungsmittel zusetzt, den hierbei entstandenen Niederschlag abtrennt, die verbleibende Lösung mit einem Kationenaustauscher behandelt, nach Abtrennung vom Kationenaustauscher leicht siedende Anteile abdestilliert und den im wesentlichen die niedermolekularen Polyhydroxylverbindungen enthaltenden Sumpf der Destillation mit einem Anionenaustauscher behandelt.

Als wässrige Lösungen von niedermolekularen Polyhydroxylverbindungen seien beispielsweise solche genannt, die 10 bis 80, bevorzugt 40 bis 75 Gew.% niecermolekulare Polyhydroxylverbindungen mit 2 bis 10, in der Hauptsache 2 bis 6 Kohlenstoffatomen und 0,005 bis 5, bevorzugt 0,01 bis 1 Gew.% Calcium- und/oder Bleiionen enthalten.

Die Menge Methanol, die der wässrigen Lösung der niedermolekularen Polyhydroxylverbindungen im erfindungsgemässen Verfahren zugefügt wird, kann 20 bis 300 Gew.%, bezogen auf die Menge der wässrigen Lösung der niedermolekularen Polyhydroxylverbindungen, betragen. Die bevorzugt zugefügte Menge Methanol beträgt 100 bis 200 Gew.%, bezogen auf die Menge der wässrigen Lösung der niedermolekularen Polyhydroxylverbindungen.

Zur Ausfällung der in der Lösung der niedermolekularen Polyhydroxylverbindungen vorhandenen Calcium- und/oder Bleiverbindungen wird anschliessend eine äquivalente Menge Fällungsmittel zugesetzt. Als Fällungsmittel können Mittel eingesetzt werden, die mit den vorhandenen Calcium- und/oder Bleiverbindungen einen schwerlöslichen Niederschlag bilden, beispielsweise Phosphate, Oxalate, oder Sulfate, bevorzugt werden Mittel eingesetzt, die als schwerlösliche Niederschläge die Sulfate ergeben.

Zur Erreichung einer möglichst weitgehenden Ausfällung der Calcium- und/oder Bleiverbindungen kann beispielsweise ein pH-Wert von 1 bis 4, bevorzugt von 2 bis 3, in der Lösung der nieder-

molekularen Polyhydroxylverbindungen einge-stellt werden. Daher kann das Fällungsmittel zur Erzielung von beispielsweise Phosphaten, Oxalaten oder Sulfaten die entsprechende Säure, wie Orthophosphorsäure, Oxalsäure oder Schwefelsäure, sein. Bevorzugt ist die Verwendung von Schwefelsäure als Fällungsmittel. Die Säure kann in konzentrierter oder verdünnter wässriger Form eingesetzt werden.

Nach der Entfernung des durch das Fällungsmittel entstandenen Niederschlages, beispielsweise durch Filtration, wird die Lösung der niedermolekularen Polyhydroxylverbindungen erfindungsgemäss mit einem Kationenaustauscher behandelt. Als Kationenaustauscher seien beispielsweise synthetische Austauscher auf der Basis von bakelit- oder aminoplastartigen Kondensationsharzen oder vernetzten Polymerisationsharzen, wie vernetztem Polystyrol, genannt, die beispielsweise Sulfonsäure-, Phosphonsäure- oder Carbonsäuregruppen tragen können (Houben-Weyl, «Methoden der organischen Chemie», Band I/1, Georg Thieme Verlag, Stuttgart 1958, Seite 527 bis 529).

Nach der Abtrennung der Lösung der niedermolekularen Polyhydroxylverbindungen vom Kationenaustauscher werden erfindungsgemäss leichtsiedende Anteile aus der Lösung abdestilliert. Als leichtsiedender Anteil seien beispielsweise genannt: Eine Teilmenge des im erfindungsgemässen Verfahren zugefügten Methanols oder leichtsiedende Anteile, die sich bei der Durchführung des erfindungsgemässen Verfahrens, gegebenenfalls im Kontakt mit dem Kationenaustauscher, gebildet haben, wie Ameisensäuremethylester. Zum erfindungsgemässen Abdestillieren leichtsiedender Anteile kann man beispielsweise eine Menge Destillat auffangen, die etwa 2 bis 15, bevorzugt 4 bis 10, Vol.% beträgt, bezogen auf das Volumen des eingesetzten Methanols.

Nach dem Abdestillieren leichtsiedender Anteile wird die Lösung der niedermolekularen Polyhydroxylverbindungen erfindungsgemäss mit einem Anionenaustauscher behandelt. Als Anionenaustauscher seien beispielsweise synthetische Austauscher auf der Basis von bakelit- oder aminoplastartigen Kondensationsharzen oder vernetzten Polymerisationsharzen, wie vernetzten Polystyrol, genannt, die beispielsweise Amino- oder Iminogruppen oder quartäre Ammoniumgruppen tragen können (Houben-Weyl, loc. cit.).

Die Lösung der niedermolekularen Polyhydroxylverbindungen kann mit dem Kationenaustauscher bzw. dem Anionenaustauscher beispielsweise dadurch behandelt werden, dass man den granulierten Austauscher in die Lösung einrührt oder die Lösung durch eine Säule leitet, die mit dem Austauscher gefüllt ist. Die bevorzugte Arbeitsweise ist das Säulenverfahren.

Die Lösung der niedermolekularen Polyhydroxylverbindungen kann beispielsweise bei einer Temperatur von 0 bis 70°C, bevorzugt 20 bis 40°C, mit dem Kationenaustauscher bzw. Anionenaustauscher behandelt werden.

Das erfindungsgemässe Verfahren kann beispielsweise wie folgt durchgeführt werden:

Zu der Lösung der niedermolekularen Polyhydroxylverbindungen wird Methanol und anschliessend eine den zu entfernenden Ionen äquivalente Menge Fällungsmittel unter Rühren zugesetzt. Anschliessend wird der entstandene Niederschlag abfiltriert und das Filtrat durch eine Säule, die mit einem Kationenaustauscher gefüllt ist, geleitet. Die von der Kationenaustauschersäule abgelaufene Lösung wird sodann in einer Destillationsapparatur solange auf etwa 60 bis 70°C erwärmt, bis eine Destillatmenge entstanden ist, die etwa 5 bis 15 Vol.% beträgt, bezogen auf das Volumen des zur Ausgangslösung zugefügten Methanols. Der Sumpf der Destillation wird auf etwa 30 bis 40°C abgekühlt und dann durch eine Säule geleitet, die mit einem Anionenaustauscher gefüllt ist, als deren Ablauf die erfindungsgemäss entionisierte Lösung von niedermolekularen Polyhydroxylverbindungen aufgefangen wird.

Das erfindungsgemässe Verfahren ermöglicht eine weitgehende Entionisierung von Lösungen niedermolekularer Polyhydroxylverbindungen. Diese Entionisierung ist vollständiger als sie nach den dem Fachmann bekannten Verfahren, wie der alleinigen Ausfällung der Ionen als unlösliche Salze oder der Abscheidung ionischer Verbindungen mit Methanol, zu erzielen ist. So ist die Löslichkeit von Calciumsulfat und Bleisulfat in Lösungen niedermolekularer Polyhydroxylverbindungen relativ hoch: 1 l wässrige Lösung etwa 60 Gew.% niedermolekularer Polyhydroxylverbindungen enthält nach der Fällung mit Schwefelsäure und Filtration noch etwa 1600 ppm Calcium bzw. etwa 27 ppm Blei.

Die nach dem erfindungsgemässen Verfahren gereinigten Lösungen niedermolekularer Polyhydroxylverbindungen enthalten weniger als 5 ppm, bevorzugt weniger als 2 ppm Calcium-Ionen und/oder weniger als 1 ppm, bevorzugt weniger als 0,5 ppm Bleiionen.

Die Abscheidung von anorganischen Kationen als schwerlösliche Salze aus Lösungen von niedermolekularen Polyhydroxylverbindungen durch den Zusatz von Methanol ist ebenfalls nur unbefriedigend, da diese Lösungen von ihrer Herstellung durch Formaldehydkondensation Ameisensäure enthalten und beispielsweise Bleiformiat auch bei Methanolzusatz noch ausreichend löslich ist.

Eine Entionisierung durch Ionenaustauscher allein würde unwirtschaftlich grosse Ionenaustauscheranlagen bedingen. Bei kleineren Ionenaustauscheranlagen müsste wegen der hohen Ionenkonzentration von Lösungen niedermolekularer Polyhydroxylverbindungen, die durch Formaldehydkondensation in Gegenwart von Calcium- bzw. Bleisalzen hergestellt worden sind, eine häufige Regenerierung der Austauscherharze vorgenommen werden. Hierbei entsteht durch das Auswaschen der am Harz haftenden Polyhydroxylverbindungen eine unerwünschte Verdünnung der Lösungen dieser Polyhydroxylverbindungen. Weiterhin ist durch die häufige Regenerierung mit einem Anfall grosser Abwassermengen zu rechnen. Die aus der Formaldehydkondensation stammende Ameisensäure würde weiterhin die Anionenaustauscher einer Ionenaustauscheranlage sehr schnell sättigen.

Es ist überraschend, dass das erfindungsgemässe Verfahren eine weitergehende Entionisierung ermöglicht, als dies aus der Aneinanderreihung der oben geschliderten, dem Fachmann bekannten Verfahren zu erwarten war. Weiter ist es überraschend, dass eine Veresterung der in den Lösungen von niedermolekularen Polyhydroxylverbindungen enthaltenden Ameisensäure mit Methanol möglich ist, obwohl diese Lösungen einen grossen molaren Überschuss an Wasser enthalten und dass hierdurch die Entfernung der Ameisensäure als Methylformiat möglich wird, so dass die Ameisensäure den Anionenaustauscher nicht zu schnell sättigt.

*Beispiel 1 (Vergleichsbeispiel)*

10 kg einer wässrigen Lösung, die 58,6 Gew.% niedermolekulare Polyhydroxylverbindungen enthält und ausserdem 7000 ppm Calciumionen, 860 ppm Blei-(II)-Ionen sowie 1,27% Formiationen (Rest Wasser) wird bei Raumtemperatur unter Rühren mit 600 ml 20,1 gew.%iger Schwefelsäure versetzt. Die Lösung wird von dem entstandenen Niederschlag abfiltriert. Man erhält 176 g Filterrückstand. Im Filtrat wurden ermittelt: 1600 ppm Ca-Ionen, 27 ppm Pb-Ionen, 1,12 Gew.% Formiationen und 0,32 Gew.% Sulfationen.

*Beispiel 2 (Vergleichsbeispiel)*

10 kg einer Lösung wie im Beispiel 1 werden zunächst mit 1830 g Äthylenglykol und danach mit 600 ml 20,1 gew.%iger Schwefelsäure unter Rühren bei Zimmertemperatur versetzt. Der gebildete Niederschlag wird abfiltriert. Man erhält einen Filterrückstand von 191 g. Im Filtrat wurden nachgewiesen: 1100 ppm Ca-Ionen, 25 ppm Pb-Ionen, 0,86 Gew.% Formiationen und 0,22 Gew.% Sulfationen.

*Beispiel 3 (erfindungsgemäss)*

10 kg Lösung wie im Beispiel 1 werden zunächst mit 15 kg Methanol und dann mit 600 ml 20,1 gew.%iger Schwefelsäure unter Rühren bei Raumtemperatur versetzt. Der entstandene Niederschlag wird abfiltriert. Man erhält einen Filterrückstand von 232 g. Im Filtrat wurden nachgewiesen: 41 ppm Ca-Ionen, 5,0 ppm Pb-Ionen, 0,24 Gew.% Formiationen und 0,032 Gew.% Sulfationen.

*Beispiel 4 (Vergleichsbeispiel)*

Zu 1 kg voll entsalzter wässriger Lösung mit 60 Gew.% niedermolekularen Polyhydroxylverbindungen werden 600 g Methanol, 24,1 g basisches Bleicarbonat (=19,28 g Blei) und 6 g Ameisensäure gegeben. Diese Mischung wird 2 Stunden bei 70°C gerührt und dann filtriert. Im klaren Filtrat wird eine Gesamtbleimenge von 17,485 g gefunden. Das Filtrat wird durch Vakuumdestillation von Wasser und Methanol befreit. Der hellgelbe, klare Rückstand enthält 17,1 g Gesamtblei. Dieser Rückstand wird 2 mal mit der doppelten Menge an Methanol gelöst und wieder eingedampft. Aus den methanolischen Lösungen schied sich hierbei kein Bleiformiat ab. Zum Schluss werden im Gemisch der niedermolekularen Polyhydroxylverbindungen noch 16,8 g Blei gefunden. Die Differenz der eingebrachten Bleimenge von 17,485 g und der zuletzt gefundenen Bleimenge von 16,8 g wurde mit den Analysenproben entnommen.

*Beispiel 5*

Je 1000 g wässrige Lösung mit 60 Gew.% niedermolekularen Polyhydroxylverbindungen und einem Gehalt von 0,7 Gew.% Ameisensäure werden mit den in der Tabelle 1 angegebenen Mengen Methanol versetzt und durch eine Säule, die mit einem stark sauren Kationenaustauscher beschickt ist, geleitet. Die Tabelle 1 zeigt die jeweils durch Titration ermittelten Gehalte an Ameisensäure vor dem Kationenaustauscher und nach dem Kationenaustauscher.

Tabelle 1

| Lösung der niedermol. Poly- hydroxyl- verbindung g | Methanol- zusatz g | Ameisensäuregehalt | |
|---|---|---|---|
| | | vor Aus- tauscher % | nach Aus- tauscher % |
| 1000 | 200 | 0,58 | 0,213 |
| 1000 | 400 | 0,516 | 0,183 |
| 1000 | 600 | 0,459 | 0,136 |
| 1000 | 1000 | 0,364 | 0,119 |
| 1000 | 1300 | 0,318 | 0,072 |
| 1000 | 1600 | 0,29 | 0,055 |
| 1000 | 1900 | 0,26 | 0,040 |

*Beispiel 6*

Je 1000 g Lösung wie im Beispiel 5, jedoch mit einem Gehalt von 3,4 Gew.% Ameisensäure werden wie in Beispiel 5 behandelt. Die Ergebnisse sind in der Tabelle 2 zusammengestellt.

Tabelle 2

| Lösung der niedermol. Poly- hydroxyl- verbindung g | Methanol- zusatz g | Ameisensäuregehalt | |
|---|---|---|---|
| | | vor Aus- tauscher % | nach Aus- tauscher % |
| 1000 | 200 | 2,79 | 0,694 |
| 1000 | 400 | 2,36 | 0,637 |
| 1000 | 600 | 2,0 | 0,373 |
| 1000 | 1000 | 1,7 | 0,274 |
| 1000 | 1300 | 1,4 | 0,185 |
| 1000 | 1600 | 1,3 | 0,168 |
| 1000 | 1900 | 1,2 | 0,150 |

*Beispiel 7*

2500 g einer wässrigen Lösung, die 1500 g = 60,0 Gew.% niedermolekulare Polyhydroxylverbindungen, 7226 mg Calciumionen, 83 mg Blei-(II)-Ionen und 16,2 g Formiationen enthält, wird bei Raumtemperatur unter Rühren mit 5000 g Methanol versetzt. Anschliessend wird diese Lösung ebenfalls unter Rühren bei Raumtemperatur mit 78 ml 20 gew.%iger Schwefelsäure versetzt. Der ausgefallene Niederschlag wird abgesaugt. Man erhält 30 g Filterrückstand, dessen Analyse einen Gehalt von 21,4 Gew.% Calcium, 2,4 Gew.% Blei und 53,1 Gew.% Sulfationen anzeigt. Das Filtrat enthält 130 ppm Calciumionen, 750 ppb Blei- (II)-Ionen, 0,013 Gew.% Sulfationen und 0,18 Gew.% Formiationen. Dieses Filtrat wird sodann bei Raumtemperatur und mit einer Durchsatzmenge von 11,7 Liter pro Stunde durch eine Chromatographiersäule geschickt, die mit 41 Litern eines stark sauren (Sulfonsäuregruppen enthaltenden) Karionenaustauschers auf der Basis eines mit Divinylbenzol vernetzten Polystyrols enthält. Der Ablauf aus dieser Chromatographiersäule enthält 1 ppm Calciumionen, weniger als 50 ppb Blei-(II)-Ionen, 0,015 Gew.% Sulfationen und 0,02 Gew.% Formiationen.

Diese als Ablauf erhaltene Lösung wird in einer Destillationsapparatur auf etwa 65°C erwärmt, wobei eine Destillatmenge abgenommen wird, die 10% der oben beschriebenen Methanolmenge entspricht.

Der Destillationsrückstand wird dann auf etwa 35°C abgekühlt. Seine Analyse zeigt 1 ppm Calciumionen, weniger als 50 ppb Blei-(II)-Ionen, 0,015 Gew.% Sulfationen und weniger als 0,02 Gew.% Formiationen an. Dieser Destilla- tionsrückstand wird sodann bei etwa 35°C und mit einer Durchsatzmenge von 0,65 Liter pro Stunde über eine Chromatographiersäule geschickt, die mit 1 Liter eines makroporösen, schwachbasi- schen (Aminogruppen enthaltenden) Anionen- austauschers auf der Basis eines durch Divinylbenzol vernetzten Polystyrols gefüllt ist. Der Ablauf der Chromatographiersäule mit dem Anionenaustauscher enthält 1 ppm Calciumionen, weniger als 50 ppb Blei-(II)-Ionen, 0,06 Gew.% Sulfa- tionen und keine nachweisbare Menge an Formiationen.

*Beispiel 8* (Herstellung der Rohformose)

Einem Formaldehydreaktor wird 45 m³ Formaldehydprozessgas/h entnommen. Diese 45 m³ enthalten bei folgender Zusammensetzung 4950 kg Formaldehyd.

| Zusammen-setzung: | | |
|---|---|---|
| $CH_2O$ | ~16-17,0 | Vol. % |
| $H_2O$ | ~41-43,0 | Vol. % |
| $CH_3OH$ | ~0,7 | Vol. % |
| $CH_4$ | ~0,06 | Vol. % |
| $HCOOCH_3$ | ~0,06 | Vol. % |
| CO | ~0,1 | Vol. % |
| $CO_2$ | ~1,5 | Vol. % |
| $H_2$ | ~6,4 | Vol. % |
| $N_2$ | ~31-33,0 | Vol. % |

Das Formaldehydprozessgas tritt mit einer Temperatur von ca. 120°C zusammen mit 4,8 kg Formaldehyd 30 Gew.% (=1,442 kg Formaldehyd 100%) von Raumtemperatur in einen Absorptionsreaktor ein. (Gesamtformaldehydeinsatz = 6,392 kg/h). Zuvor waren als Katalysator-Cokatalysatorgemisch für die Formosereaktion ca. 30 l einer ca. 30%igen Rohformose mit einem Formaldehydgehalt von ca. 5% und einem Calciumhy- droxydgehalt von 1,1 kg mit 15 g Bleiacetathydrat in diesem Absorptionsreaktor vorgelegt und durch Umpumpen über einen Wärmeaustauscher auf 80°C aufgeheizt worden. Durch das heisse Formaldehydprozessgas steigt die Temperatur der vorgelegten Formose auf 89°C an und die Umsetzung des Formaldehyds wird durch mässige exotherme Reaktion angezeigt. Trotz erheblicher Wärmeabstrahlung der nicht isolierten Apparatur hält sich die Temperatur im Sumpf des Absorptionsturmes bei 89°C fast ohne Wärmezufuhr über den Wärmeaustauscher. Gleichzeitig mit der Formaldehydzugabe werden stündlich 0,11 kg Calciumhydroxid als 10%ige Suspension und 8 g Bleiacetathydrat als 2%ige Lösung in den Reaktor eingespeist. Nach 5 bis 8 Stunden hat sich in der Apparatur ein Gleichgewichtszustand eingestellt. Das drückt sich durch konstante Abnahmemengen und Konzentrationen aus. Die genannte Menge 30%iger Formaldehydlösung führt hierbei zu einer etwa 60 gew.%igen Formoselösung, die am Überlauf des Absorptionsreaktors abgenommen wird. Die produzierte Formose ist von reproduzierbarer Zusammensetzung; bei einer mittleren Funktionalität von 5,66 OH-Gruppen pro Molekül ent- hält sie: 1,1% $C_2$, 3,6% $C_3$, 5,3%/ $C_4$, 13,7% $C_5$, 43,7% $C_6$, 28,1% $C_7$, 4,5% $C_8$. Bei einer Versuchs- dauer von 120 Stunden konnte eine Gesamtausbeute von 95,3%, bezogen auf eingesetztes Formaldehyd erreicht werden. Die dem Absorptions- reaktor entnommene Formose enthält durch- schnittlich 7000 ppm Calcium- und etwa 850 ppm Bleiionen sowie 1,25-1,3 Gew.% Formiationen. Geringere Mengen an Calciumhydroxid und Bleiacetat führen zu einer längeren Reaktionszeit und geringeren Ausbeuten.

**Patentansprüche**

1. Verfahren zur Reinigung von wässrigen Lö- sungen von niedermolekularen Polyhydroxyl- verbindungen, die Calcium- oder Bleiverbindun- gen oder ein Gemisch von Calcium- und Bleiverbindungen neben Ameisensäure enthalten, durch Entfernung von Calcium- und/oder Bleiverbindungen, sowie der Ameisensäure durch Mit- verwendung von Ionenaustauscher- und Fäl- lungsmethoden, dadurch gekennzeichnet, dass man diesen Lösungen Methanol zufügt, eine den zu entfernenden Ionen äquivalente Menge Fällungsmittel zusetzt, den hierbei entstandenen Niederschlag abtrennt, die verbleibende Lösung mit einem Kationenaustauscher behandelt, nach Ab- trennung vom Kationenaustauscher leicht sieden- de Anteile abdestilliert und den im wesentlichen die niedermolekularen Polyhydroxylverbindungen enthaltenden Sumpf der Destillation mit einem Anionenaustauscher behandelt.

2. Verfahren nach Anspruch 1, dadurch gekennzeichnet, dass man Methanol in einer Menge von 20 bis 300 Gew.%, bezogen auf die Menge der wässrigen Lösung der niedermolekularen Polyhydroxylverbindungen, zufügt.

3. Verfahren nach Anspruch 1 und 2, dadurch gekennzeichnet, dass man als Fällungsmittel Phosphate, Oxalate oder Sulfate zusetzt.

4. Verfahren nach Anspruch 1 bis 3, dadurch gekennzeichnet, dass man als Fällungsmittel Orthophosphorsäure, Oxalsäure oder Schwefelsäure zusetzt.

5. Verfahren nach Anspruch 1 bis 4, dadurch gekennzeichnet, dass man zur Ausfällung einen pH-Wert von 1 bis 4 einstellt.

6. Verfahren nach Anspruch 1 bis 5, dadurch gekennzeichnet, dass der Niederschlag durch Filtration abgetrennt wird.

7. Verfahren nach Anspruch 1 bis 6, dadurch gekennzeichnet, dass zur Behandlung mit dem Kationenaustauscher und/oder mit dem Anionenaustauscher jeweils der granulierte Austauscher in die Lösung eingerührt wird.

8. Verfahren nach Anspruch 1 bis 6, dadurch gekennzeichnet, dass zur Behandlung mit dem Kationenaustauscher und/oder mit dem Anionenaustauscher die Lösung durch eine Säule geleitet wird, die mit dem jeweiligen Austauscher gefüllt ist.

9. Verfahren nach Anspruch 1 bis 8, dadurch gekennzeichnet, dass die Behandlung mit dem Kationenaustauscher bzw. mit dem Anionenaustauscher bei 0° bis 70°C erfolgt.

10. Verfahren nach Anspruch 1 bis 9, dadurch gekennzeichnet, dass zum Abdestillieren leichtsiedender Anteile eine Destillatmenge aufgefangen wird, die 2 bis 15 Vol% des eingesetzten Volumens Methanol beträgt.

## Claims

1. Process for the purification of aqueous solutions of low molecular weight polyhydroxyl compounds containing calcium or lead compounds or a mixture of calcium and lead compounds in addition to formic acid by removing calcium and/or lead compounds as well as the formic acid by the joint use of ion exchanger and precipitation methods, characterised in that methanol is added to these solutions, a precipitant is added in a quantity equivalent to the ions to be removed, the deposit formed is separated off, the residual solution is treated with a cation exchanger, low-boiling fractions are distilled off after separation from the cation exchanger and the distillation sump essentially containing the low molecular weight polyhydroxyl compounds is treated with an anion exchanger.

2. Process according to Claim 1, characterised in that methanol is added in an amount of 20 to 300% by weight, based on the quantity of the aqueous solution of the low molecular weight polyhydroxyl compounds.

3. Process according to Claim 1 and 2, characterised in that phosphates, oxalates or sulphates are added as precipitants.

4. Process according to Claim 1 to 3, characterised in that orthophosphoric acid, oxalic acid or sulphuric acid are added as precipitants.

5. Process according to Claim 1 to 4, characterised in that a pH value of 1 to 4 is adjusted for the precipitation.

6. Process according to Claim 1 to 5, characterised in that the deposit is separated off by filtration.

7. Process according to Claim 1 to 6, characterised in that for the treatment with the cation exchanger and/or with the anion exchanger in each case the granulated exchanger is stirred into the solution.

8. Process according to Claim 1 to 6, characterised in that for the treatment with the cation exchanger and/or with the anion exchanger the solution is passed through a column filled with the respective exchanger.

9. Process according to Claim 1 to 8, characterised in that the treatment with the cation exchanger or with the anion exchanger is carried out at 0° to 70°C.

10. Process according to Claim 1 to 9, characterised in that for the purpose of distilling off low-boiling fractions a quantity of distillate is collected which amounts to 2 to 15% by volume of the volume of methanol used.

## Revendications

1. Procédé de purification de solutions aqueuses de composés polyhydroxy de faible poids moléculaire contenant des composés de calcium ou des composés de plomb ou encore un mélange de composés de calcium et de plomb en plus de l'acide formique, par élimination de ces composés de calcium et/ou de plomb, ainsi que de l'acide formique en recourant conjointement à des procédés d'échange d'ions et de précipitation, caractérisé en ce qu'on ajoute du méthanol à ces solutions, on ajoute un agent de précipitation en une quantité équivalant à celle des ions à éliminer, on sépare le précipité ainsi formé, on traite la solution résiduelle avec un échangeur de cations, on distille les fractions d'un faible point d'ébullition après séparation de l'échangeur de cations et on traite, avec un échangeur d'anions, les queues de distillation contenant essentiellement les composés polyhydroxy de faible poids moléculaire.

2. Procédé suivant la revendication 1, caractérisé en ce qu'on ajoute le méthanol en une quantité de 20 à 300% en poids, calculés sur la quantité de la solution aqueuse des composés polyhydroxy de faible poids moléculaire.

3. Procédé suivant l'une quelconque des revendications 1 ou 2, caractérisé en ce que, comme agents de précipitation, on utilise des phosphates, des oxalates ou des sulfates.

4. Procédé suivant l'une quelconque des revendications 1 à 3, caractérisé en ce que, comme agent de précipitation, on ajoute de l'acide orthophosphorique, de l'acide oxalique ou de l'acide sulfurique.

5. Procédé suivant l'une quelconque des revendications 1 à 4, caractérisé en ce que, pour la précipitation, on règle un pH de 1 à 4.

6. Procédé suivant l'une quelconque des revendications 1 à 5, caractérisé en ce qu'on sépare le précipité par filtration.

7. Procédé suivant l'une quelconque des revendications 1 à 6, caractérisé en ce que, pour le traitement avec l'échangeur de cations et/ou avec l'échangeur d'anions, on délaie chaque fois l'échangeur granulaire dans la solution.

8. Procédé suivant l'une quelconque des revendications 1 à 6, caractérisé en ce que, pour le traitement avec l'échangeur de cations et/ou avec l'échangeur d'anions, on fait passer la solution à travers une colonne remplie de chaque échangeur.

9. Procédé suivant l'une quelconque des revendications 1 à 8, caractérisé en ce qu'on effectue le traitement avec l'échangeur de cations ou avec l'échangeur d'anions à une température de 0 à 70°C.

10. Procédé suivant l'une quelconque des revendications 1 à 9, caractérisé en ce que, pour séparer les fractions d'un faible point d'ébullition par distillation, on recueille le distillat en une quantité représentant 2 à 15% du volume de méthanol utilisé.